# FASCICULE DE BREVET EUROPEEN

(11) **EP 3 442 631 B1**
(45) Date de publication et mention de la délivrance du brevet: **29.04.2020**
(21) Numéro de dépôt: 17723446.5
(22) Date de dépôt: 13.04.2017
(51) Int. Cl.: A61M 16/00, A61M 15/00

(54) **DISPOSITIF DE DISTRIBUTION DE PRODUIT FLUIDE SYNCHRONISE AVEC L'INHALATION**
VORRICHTUNG ZUR INHALATIONSSYNCHRONISIERTEN AUSGABE EINES FLÜSSIGPRODUKTS
DEVICE FOR INHALATION-SYNCHRONISED DISPENSING OF A FLUID PRODUCT

(30) Priorité: 15.04.2016 FR 1653367
(43) Date de publication de la demande: 20.02.2019
(73) Titulaire: Aptar France SAS, 27110 Le Neubourg (FR)
(72) Inventeur: PETIT, Ludovic, 27110 Vitot (FR)
(74) Mandataire: CAPRI
(86) Numéro de dépôt international: PCT/FR2017/050891
(87) Numéro de publication internationale: WO 2017/178765

(56) Documents cités:
- EP-A1- 0 441 643
- WO-A1-85/01880
- DE-A1- 3 040 641
- FR-A1- 2 775 668
- US-A- 5 060 643

## Description

La présente invention concerne un dispositif de distribution de produit fluide synchronisé avec l'inhalation, et plus particulièrement un dispositif d'inhalation du type aérosol synchronisé avec l'inhalation.

Les dispositifs actionnés par l'inhalation, désignés généralement par le terme B.A.I. (signifiant "Breath Actuated Inhaler"), sont bien connus dans l'état de la technique. L'avantage principal de ce type de dispositif est que la distribution du produit est synchronisée avec l'inhalation du patient, pour garantir une bonne distribution du produit dans les voies respiratoires. Ainsi, dans le domaine des dispositifs aérosol, c'est-à-dire ceux dans lesquels le produit est distribué à l'aide d'un gaz propulseur, de nombreux types de dispositifs de déclenchement par l'inhalation ont été proposés. Ces dispositifs présentent toutefois l'inconvénient de comporter un grand nombre de pièces, c'est-à-dire qu'ils sont compliqués et coûteux à fabriquer et à assembler, ce qui est évidemment désavantageux. Il est aussi difficile de trouver le bon équilibre entre un déclenchement fiable à chaque inhalation, sans que le seuil de déclenchement ne soit trop élevé, et une serrure suffisamment robuste pour empêcher un actionnement accidentel ou non souhaité. Or, si la serrure se déverrouille de manière accidentelle, le dispositif est actionné automatiquement et la dose est distribuée, même si l'utilisateur ne l'a pas souhaité.

Ainsi, plus que l'actionnement automatique du dispositif, ce qui est important pour obtenir une bonne distribution de la dose, c'est de réaliser cette distribution de manière synchronisée avec l'inhalation de l'utilisateur, même si l'actionnement ou le déclenchement proprement dit reste manuel.

Les documents FR2775668 et US5060643 décrivent des dispositifs de l'état de la technique.

La présente invention a pour but de fournir un dispositif de distribution de produit fluide synchronisé avec l'inhalation qui ne reproduit pas les inconvénients susmentionnés.

La présente invention a aussi pour but de fournir un dispositif de distribution de produit fluide synchronisé avec l'inhalation qui améliore la fiabilité de fonctionnement, en garantissant un actionnement efficace à chaque inhalation.

La présente invention a aussi pour but de fournir un dispositif de distribution de produit fluide synchronisé avec l'inhalation qui minimise les risques d'actionnement accidentel ou non souhaité.

La présente invention a également pour but de fournir un dispositif de distribution de produit fluide synchronisé avec l'inhalation qui ne présente pas un seuil de déclenchement trop élevé, permettant ainsi à des personnes relativement faibles, telles que des personnes malades ou âgées, d'utiliser le dispositif de manière sûre et fiable.

La présente invention a également pour but de fournir un dispositif de distribution de produit fluide synchronisé avec l'inhalation qui soit simple et peu coûteux à fabriquer et à assembler.

La présente a donc pour objet un dispositif de distribution de produit fluide synchronisé avec l'inhalation, comportant un corps pourvu d'un embout buccal, un réservoir de produit contenant un produit fluide et un gaz propulseur étant monté axialement coulissant dans ledit corps, une valve doseuse comportant une soupape étant assemblée sur ledit réservoir pour distribuer sélectivement le produit fluide, ledit dispositif comportant:
- un élément d'actionnement déplaçable et/ou déformable entre une position de non actionnement, dans laquelle ladite valve doseuse ne peut pas être actionnée, et une position d'actionnement, dans laquelle ladite valve doseuse peut être actionnée, et
- un système de déclenchement commandé par l'inhalation comportant un organe sensible à l'inhalation, déformable et/ou déplaçable sous l'effet de l'inhalation, ledit organe sensible à l'inhalation, lorsqu'il se déforme et/ou se déplace, déplaçant et/ou déformant ledit élément d'actionnement de sa position de non actionnement vers sa position d'actionnement, ledit élément d'actionnement étant un élément de blocage qui, en position de non actionnement, coopère d'une part avec le corps et d'autre part avec le réservoir pour empêcher le déplacement axial dudit réservoir dans le corps.

Avantageusement, ledit élément d'actionnement est une bague de blocage fixée, notamment encliquetée, sur ledit réservoir et comportant au moins une patte axiale, notamment trois, coopérant avec un épaulement solidaire dudit corps pour bloquer le déplacement axial dudit réservoir dans ledit corps.

Avantageusement, ladite au moins une patte axiale est déformable radialement vers l'extérieur pour passer de la position de non actionnement vers la position d'actionnement, un élément déclencheur étant prévu pour maintenir ladite au moins une patte axiale en position de non actionnement.

Avantageusement, ledit élément déclencheur est monté déplaçable entre une position de blocage, dans laquelle il bloque ladite bague de blocage dans sa position de non actionnement, et une position de libération, dans laquelle il ne bloque pas ladite bague de blocage.

Avantageusement, ledit système de déclenchement commandé par l'inhalation comporte une membrane déformable définissant une chambre d'air déformable, ladite membrane déformable étant fixée audit élément déclencheur, ladite membrane déformable étant déformée lors de l'inhalation de telle sorte qu'elle déplace ledit élément déclencheur de sa position de blocage vers sa position de libération.

Avantageusement, ledit élément déclencheur est accessible par l'utilisateur à travers au moins une ouverture du corps, pour pouvoir être déplacé manuellement vers sa position de libération même en l'absence d'inhalation.

Avantageusement, ledit élément de blocage comporte une extension axiale dont une extrémité inférieure est fixée radialement et axialement par rapport audit corps et une extrémité supérieure coopère avec ledit réservoir en position de non actionnement.

Avantageusement, ledit système de déclenchement commandé par l'inhalation comporte un piston coulissant dans une chambre entre une position de repos et une position d'inhalation.

Avantageusement, ledit élément de blocage est assemblé sur une tige solidaire du piston, de sorte que lors de l'inhalation, ladite tige se déplace radialement déformant et/ou déplaçant ladite extension axiale vers sa position d'actionnement.

Avantageusement, ledit dispositif comporte un compteur de doses électronique.

Avantageusement, ledit dispositif comporte des moyens d'émission de signaux pour communiquer à distance notamment des informations relatives aux actionnements du dispositif.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en section d'un dispositif de distribution de produit fluide, selon un premier mode de réalisation avantageux, en position de repos,
- la figure 2 est une vue similaire à celle de la figure 1, selon un autre plan de coupe verticale,
- la figure 3 est une vue similaire à celle de la figure 1, en position de non distribution avant aspiration,
- la figure 4 est une vue similaire à celle de la figure 3, en position après aspiration,
- la figure 5 est une vue similaire à celle de la figure 2, en position de distribution d'une dose de produit fluide,
- la figure 6 est une vue similaire à celle de la figure 5, selon un autre plan de coupe verticale,
- la figure 7 est une vue similaire à celle de la figure 6, en position après distribution d'une dose de produit fluide,
- la figure 8 est une vue schématique en section d'une variante de réalisation de la chambre d'air, en position de repos,
- la figure 9 est une vue similaire à celle de la figure 8, illustrant une autre variante de réalisation intégrant de l'électronique, en position de repos,
- la figure 10 est une vue schématique de détail en perspective du corps de la figure 1,
- la figure 11 est une vue schématique de détail en perspective de la bague de blocage de la figure 1,
- la figure 12 est une vue schématique de détail en perspective de l'élément déclencheur de la figure 1,
- la figure 13 est une vue schématique de détail en perspective découpée de la membrane déformable de la figure 1,
- la figure 14 est une vue schématique découpée d'un dispositif de distribution de produit fluide, selon encore un autre mode de réalisation avantageux, en position de repos,
- la figure 15 est une vue similaire à celle de la figure 14, en position de distribution, et
- la figure 16 est une vue similaire à celle de la figure 14, illustrant une variante de réalisation.

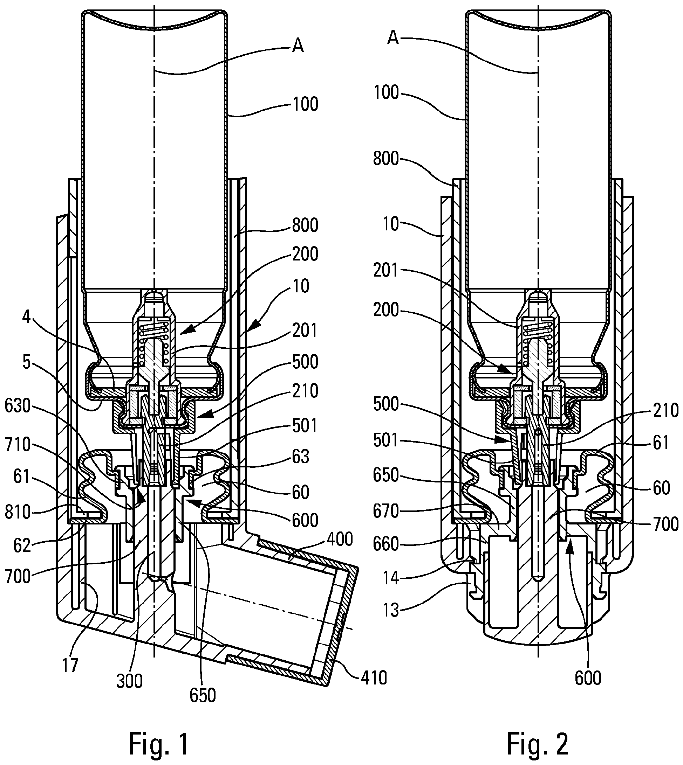

Dans la description, les termes "supérieur", "inférieur", "haut" et "bas" se réfèrent à la position du dispositif représentée notamment sur la figure 1. Les termes "axial" et "radial" se réfèrent à l'axe central vertical A représenté notamment sur la figure 1. Les termes "proximal" et "distal" se réfèrent à l'embout buccal.

L'invention s'applique plus particulièrement à des dispositifs d'inhalation du type à valve aérosol pour une distribution orale, comme cela sera décrit plus en détail ci-après, mais elle pourrait aussi s'appliquer à d'autres types de dispositifs d'inhalation, par exemple du type nasal.

Les figures représentent plusieurs modes de réalisation avantageux de l'invention, mais il est entendu que l'une ou plusieurs des pièces constitutives décrites ci-après pourrai(en)t être réalisée(s) différemment tout en procurant des fonctions similaires voire identiques.

En référence aux dessins, le dispositif comporte un corps principal 10 pourvu d'un embout buccal 400. Cet embout buccal 400 définit un orifice de distribution à travers lequel l'utilisateur va inhaler lors de l'utilisation du dispositif. Un capot de protection amovible 410 peut être prévu sur ledit embout buccal 400, notamment lors des périodes de stockage, que l'utilisateur va retirer avant utilisation. Les figures 1, 8 et 9 montrent un tel capot de protection, qui pourrait être de forme quelconque.

Le corps 10 contient un réservoir 100, contenant le produit à distribuer et un gaz propulseur, tel qu'un gaz du type HFA, une valve doseuse 200 étant montée sur ledit réservoir 100 pour distribuer sélectivement le produit. La valve doseuse 200 comporte un corps de valve 201 et une soupape 210 axialement déplaçable lors de l'actionnement par rapport audit corps de valve 201, et donc par rapport audit réservoir 100. Cette valve doseuse 200 peut être d'un type quelconque approprié. Elle peut être fixée au réservoir 100 par un élément de fixation, de préférence une capsule sertie 5, de préférence avec interposition d'un joint de col 4.

Avantageusement, lors de l'actionnement, la soupape 210 est fixe par rapport au corps 10, et c'est le réservoir 100 qui est déplacé axialement par rapport au corps 10 entre une position distale, qui est la position de repos, et une position proximale.

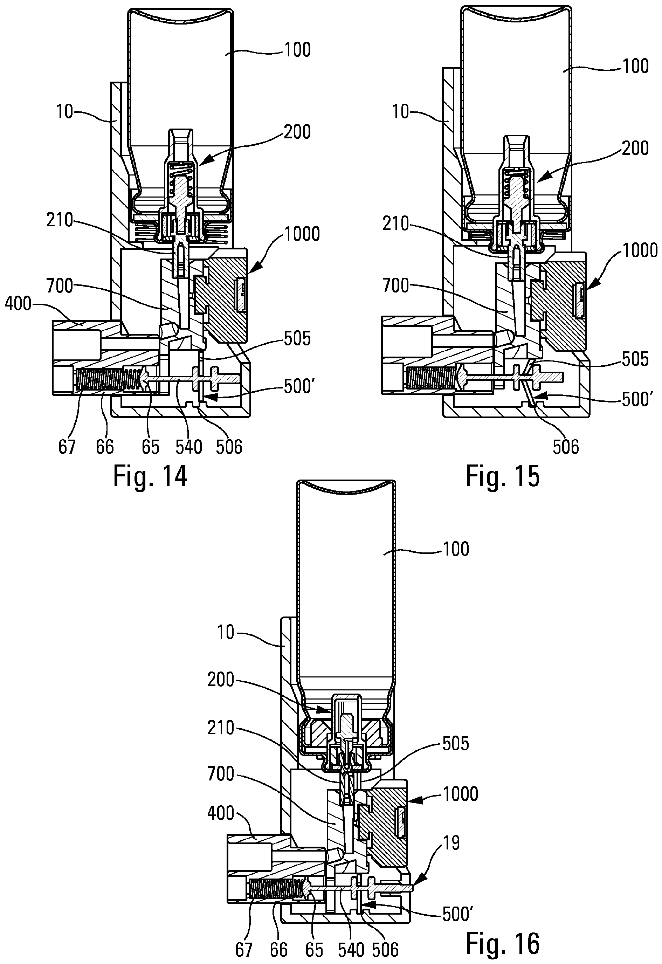

L'orifice de sortie de la soupape 210 de ladite valve doseuse 200 est relié via un canal 300 audit embout buccal 400, à travers lequel l'utilisateur inhale le produit distribué. De manière connue, ladite soupape 210 est reçue dans un puits de soupape 700 qui définit au moins partiellement ledit canal 300. Dans les modes de réalisation des figures 1 à 9, le puits de soupape est formé d'une pièce avec le corps 10, alors que dans les modes de réalisations des figures 14 à 19, ledit puits de soupape est axialement déplaçable par rapport audit corps 10.

Selon l'invention, le dispositif comporte un élément d'actionnement 500, 500' déplaçable et/ou déformable entre une position de non actionnement, dans laquelle ladite valve doseuse 200 ne peut pas être actionnée, et une position d'actionnement, dans laquelle ladite valve doseuse 200 peut être actionnée. En position de repos, ledit élément d'actionnement 500, 500' est en position de non actionnement, et c'est l'inhalation de l'utilisateur à travers l'embout buccal 400 qui déplace et/ou déforme ledit élément d'actionnement 500, 500' vers sa position d'actionnement. En d'autres termes, tant que l'utilisateur n'a pas inhalé, l'actionnement de la valve doseuse 200 est impossible, et ce n'est que lorsqu'il inhale qu'il peut actionner ladite valve doseuse 200, avantageusement par appui manuel sur le fond du réservoir 100.

Comme cela sera décrit plus en détails ci-après, l'élément d'actionnement est un élément de blocage 500, 500' qui, en position de non actionnement, empêche le déplacement axial du réservoir 100 dans le corps 10. Lors de l'inhalation, cet élément de blocage 500, 500' est déplacé et/ou déformé de telle sorte qu'il ne bloque plus le déplacement axial du réservoir 100 dans le corps 10. Ainsi, après inhalation, un tel déplacement axial du réservoir 100 provoque l'actionnement de la valve doseuse 200 et la distribution d'une dose de produit synchronisée avec cette inhalation.

Ainsi, en l'absence d'inhalation, il n'y a aucun risque qu'une dose de produit actif soit perdue par un actionnement malencontreux ou incomplet dans lequel l'utilisateur n'exercerait pas d'inhalation. L'actionnement de la valve 200 et l'expulsion d'une dose de produit fluide ne sont donc possibles que si l'utilisateur inhale et que simultanément il appuie sur le réservoir 100 pour actionner la valve 200.

Le dispositif comporte un système de déclenchement commandé par l'inhalation de l'utilisateur, qui est destiné à déplacer et/ou déformer ledit élément d'actionnement 500, 500' de sa position de non actionnement vers sa position d'actionnement, lorsque l'utilisateur inhale à travers l'embout buccal 400.

Ce système de déclenchement comporte un organe sensible à l'inhalation 60, 65, déformable et/ou déplaçable sous l'effet de l'inhalation, cet organe sensible à l'inhalation 60, 65 étant adapté, lorsqu'il se déforme et/ou se déplace, à déplacer et/ou déformer ledit élément d'actionnement 500, 500' de sa position de non actionnement vers sa position d'actionnement.

Comme cela sera décrit plus en détails ci-après, l'organe sensible à l'inhalation peut être réalisé sous la forme d'une chambre d'air déformable 60, par exemple un soufflet ou une poche déformable.

En variante, comme cela sera décrit en référence aux figures 14 à 16, l'organe sensible à l'inhalation peut être réalisé sous la forme d'un piston 65, de préférence cylindrique, coulissant dans une chambre 66, de préférence cylindrique, non déformable.

Selon un premier mode de réalisation, représenté sur les figures 1 à 13, la position de non actionnement correspond à une position de blocage du réservoir 100 dans le corps 10. Dans cette position de blocage, le réservoir 100 est empêché de se déplacer par ledit élément d'actionnement, qui ne sera libéré qu'au moment de l'inhalation.

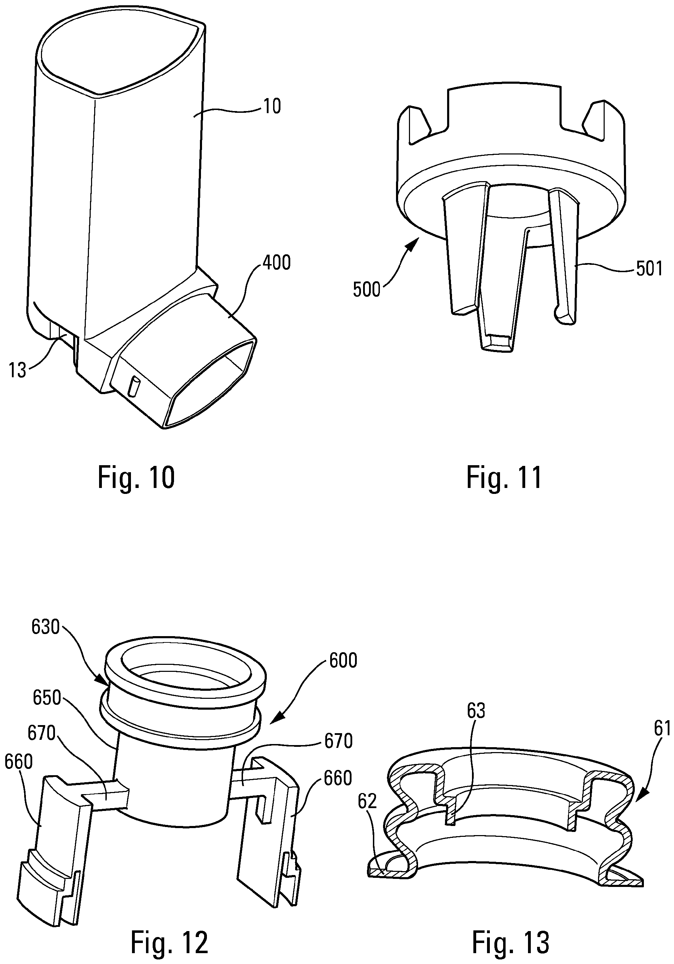

Le corps 10 est représenté notamment sur la figure 10. Bien entendu, la forme représentée n'est pas limitative.

L'élément d'actionnement, formant élément de blocage, est ici avantageusement formé par une bague de blocage 500, comportant au moins une, de préférence trois pattes axiales de blocage 501, qui sont élastiquement déformable radialement vers l'extérieur. La figure 11 représente une vue en perspective de cette bague de blocage 500. Cette bague de blocage 500 est fixée, notamment encliquetée, sur le réservoir 100, notamment sur la capsule 5 qui fixe la valve doseuse 200 sur le réservoir 100. Lesdites pattes de blocage 501 s'appuient en position de repos sur un épaulement radial 710 dudit puits de soupape 700. Cet épaulement est de préférence incliné vers le bas et radialement vers l'extérieur, de sorte que lorsque le réservoir 100 coulisse axialement dans le corps 10 lors de l'actionnement, lesdites pattes axiales de blocage 501 glissent sur ledit épaulement 710 incliné, ce qui les déforme radialement vers l'extérieur.

Un élément déclencheur 600 est monté coulissant axialement autour dudit puits de soupape 700 entre une position de blocage, dans laquelle il bloque ladite bague de blocage 500 dans sa position de non actionnement, et une position de libération, dans laquelle il ne bloque plus ladite bague de blocage 500. En particulier, dans le mode de réalisation des figures 1 à 13, ledit élément déclencheur 600, en position de blocage, coopère avec lesdites pattes de blocage 501, empêchant toute déformation radiale vers l'extérieur desdites pattes de blocage 501. Ainsi, lorsque ledit élément déclencheur 600 est en position de blocage, il empêche la déformation radiale vers l'extérieur desdites pattes de blocage 501, qui par conséquent restent axialement bloquées par ledit épaulement 710 du puits de soupape 700, ce qui bloque le déplacement axial du réservoir 100 et donc l'actionnement de la valve doseuse 200. Eventuellement, des moyens de glissement, tels que des billes, pourraient être interposées entre le puits de soupape 700 et l'élément déclencheur 600, pour faciliter le coulissement de ce dernier lors de l'inhalation.

L'élément déclencheur 600, plus particulièrement visible sur la figure 12, comporte avantageusement un manchon central creux 650 qui coulisse axialement autour du puits de soupape 700 et deux pattes axiales 660, diamétralement opposées, et chacune reliée audit manchon central 650 par une entretoise radiale respective 670. Ces pattes axiales 660 coopèrent chacune avec une ouverture respective 13 du corps 10, pour substantiellement obturer ces ouvertures 13 dans la position de blocage, et pour substantiellement ouvrir ces ouvertures 13 dans la position de libération. Ces ouvertures 13 étant fermées en début d'inhalation, le flux d'inhalation est initialement principalement transmis au système de déclenchement par l'inhalation, dans cet exemple la chambre d'air déformable 60. Ceci permet d'optimiser ce déclenchement par l'inhalation. Lorsque l'élément déclencheur 600 s'est déplacé axialement vers sa position de libération sous l'effet de l'inhalation, et donc lorsque l'utilisateur peut actionner la valve doseuse 200 pour distribuer une dose de produit fluide, les pattes axiales 660 ouvrent lesdites ouvertures 13 du corps 10, ce qui génère un appel d'air, et augmente donc le flux d'inhalation. Ceci optimise la synchronisation entre la distribution de la dose et l'inhalation de l'utilisateur, et favorise une bonne distribution de la dose dans les poumons de l'utilisateur.

Avantageusement, lesdites pattes axiales 660 sont accessibles de l'extérieur à travers lesdites ouvertures 13. Ceci permet en cas de nécessité de déplacer manuellement l'élément déclencheur 600, pour pouvoir réaliser un actionnement de la valve doseuse 200 même sans inhalation, par exemple si la personne devant recevoir la dose de produit fluide est incapable de réaliser une inhalation suffisante. Il s'agit donc d'une sécurité. En variante pour cette sécurité, on pourrait prévoir une extension axiale (non représentée) solidaire de l'élément déclencheur 600, s'étendant par exemple latéralement du réservoir 100, et accessible par l'utilisateur depuis l'extérieur du corps 10.

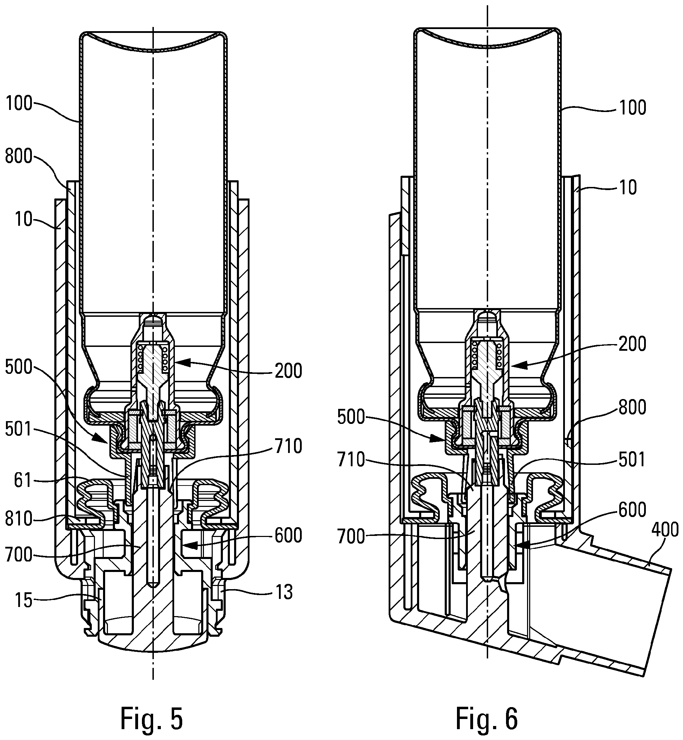

Avantageusement, comme visible sur la figure 2, lesdites pattes axiales 660 coopèrent avec un premier épaulement 14 du corps 10 pour définir la position de blocage de l'élément déclencheur 600, en formant une butée axiale qui empêche ledit élément déclencheur 600 de remonter vers le haut au-delà de cette position de blocage. De même, comme visible sur la figure 5, lesdites pattes axiales 660 coopèrent avec un second épaulement 15 du corps 10 pour définir la position de libération de l'élément déclencheur 600, en formant une butée axiale qui empêche ledit élément déclencheur 600 de descendre vers le bas au-delà de cette position de libération.

En variante (non représentée), l'élément déclencheur 600 pourrait ne pas comporter les pattes axiales 660, et le corps ne comporterait pas alors les ouvertures 13. Dans ce cas, le flux d'inhalation pourrait s'écouler axialement dans le corps, autour du puits de soupape, ce qui pourrait être favorisé par un manchon central 650 pourvu de découpes axiales permettant le passage du flux d'air.

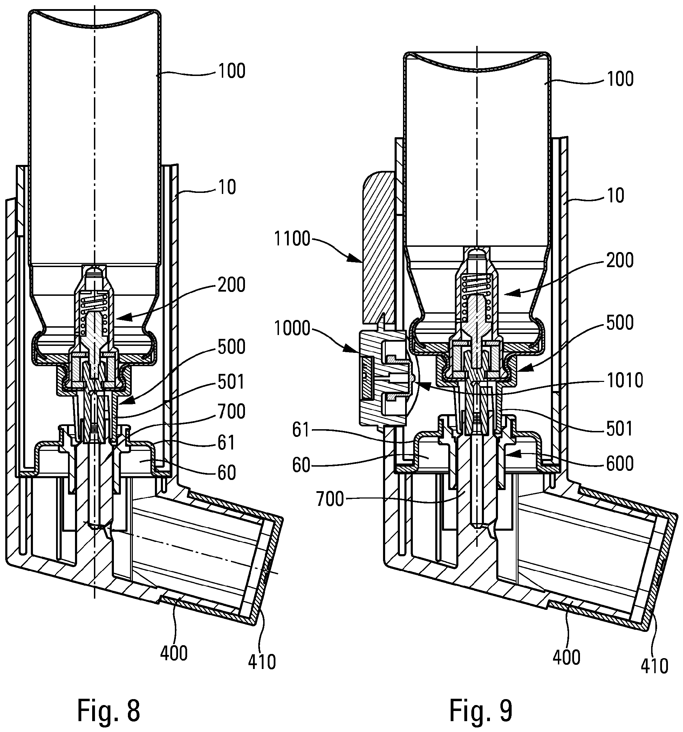

Dans le mode de réalisation des figures 1 à 13, l'organe sensible à l'inhalation 60 est réalisé sous la forme d'une chambre d'air déformable. Avantageusement, cette chambre d'air comprend une membrane déformable 61 qui est reliée d'une part au corps 10 et d'autre part audit élément déclencheur 600. Avantageusement, comme visible sur les figures 1 à 9, un manchon porte-membrane 800 est disposé fixement dans le corps 10, avec un épaulement inférieur 810 qui coince un premier bord 62 de la membrane 61 contre une partie 17 du corps 10. Le second bord 63 de la membrane 61 peut être fixé dans une gorge 630 de l'élément déclencheur 600, avantageusement formée sur le manchon central creux 650 de celui-ci. La figure 13 illustre une vue découpée d'une membrane en forme de soufflet, mais d'autres formes sont possibles, notamment une simple poche ou diaphragme, comme visible sur la figure 8.

Lors de l'inhalation, la membrane déformable 61 se déforme et/ou se contracte sous l'effet de la dépression générée par l'inhalation, provoquant le déplacement de l'élément déclencheur 600 de sa position de blocage vers sa position de libération. Ceci permet alors la déformation radiale desdites pattes de blocage 501 et donc le déplacement de ladite bague de blocage 500 formant l'élément d'actionnement de sa position de non actionnement vers sa position d'actionnement. Les figures 1 à 7 montrent une membrane déformable 61 réalisée sous la forme d'un soufflet, et la figure 8 montre une variante dans laquelle la membrane déformable est réalisée sous la forme d'une poche ou d'un diaphragme. Bien entendu, d'autres formes sont aussi envisageables.

La valve 200 n'est donc actionnée qu'au moment de l'inhalation, de sorte que la dose de produit fluide est expulsée hors de l'orifice de distribution simultanément à l'inhalation.

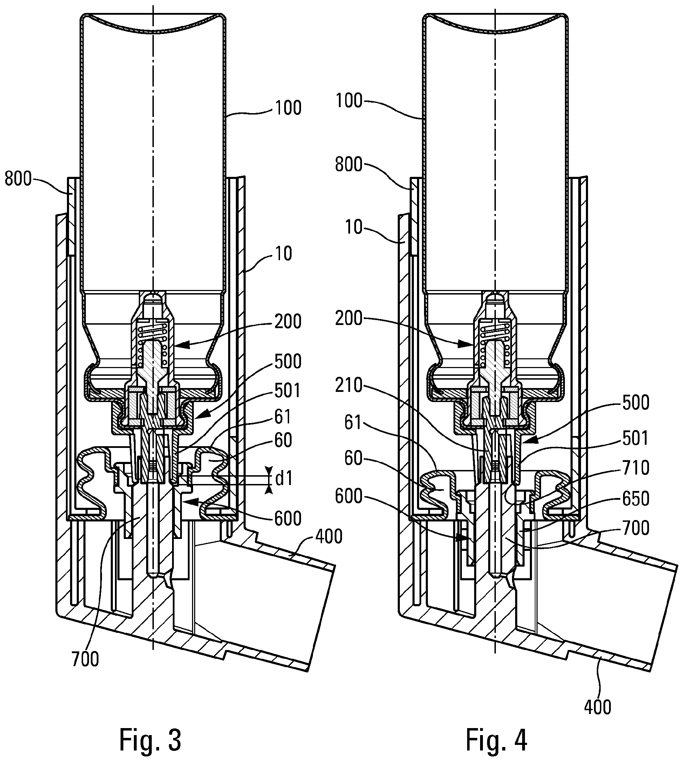

Lorsque l'utilisateur souhaite utiliser le dispositif, il place l'embout buccal 400 dans sa bouche, et appuie manuellement sur le fond du réservoir 100, c'est-à-dire la surface supérieure dudit réservoir 100 dans la position des figures. Le réservoir 100 va alors se trouver bloqué et empêché de coulisser dans le corps 10 par les pattes de blocage 501 de la bague de blocage 500, qui s'appuient sur l'épaulement 710 du puits de soupape 700. Eventuellement, le réservoir 100 peut effectuer une petite course d1 avant d'être bloqué, cette petite course initiale d1 étant toutefois insuffisante pour actionner la valve doseuse 200. La figure 1 montre le dispositif au repos et la figure 3 montre le dispositif en position de blocage du réservoir 100, après que celui-ci a réalisé cette petite course initiale d1.

Lorsque l'utilisateur inhale à travers l'embout buccal 400, il va déformer la membrane déformable 61, ce qui va faire coulisser l'élément déclencheur 600 fixé à ladite membrane déformable 61, comme visible sur la figure 4. Ce déplacement de l'élément déclencheur 600 sur le puits de soupape 700 va libérer radialement les pattes 501 de la bague de blocage 500. Sous l'effet de la force axiale transmise par le réservoir 100, générée par l'utilisateur qui appuie sur le fond dudit réservoir 100, les pattes axiales 501 vont pouvoir se déformer radialement vers l'extérieur, et ainsi passer par-dessus ledit épaulement 710, pour permettre le coulissement du réservoir 100 vers sa position de distribution, et donc l'actionnement de la valve 200. Cette position de distribution est représentée sur les figures 5 et 6.

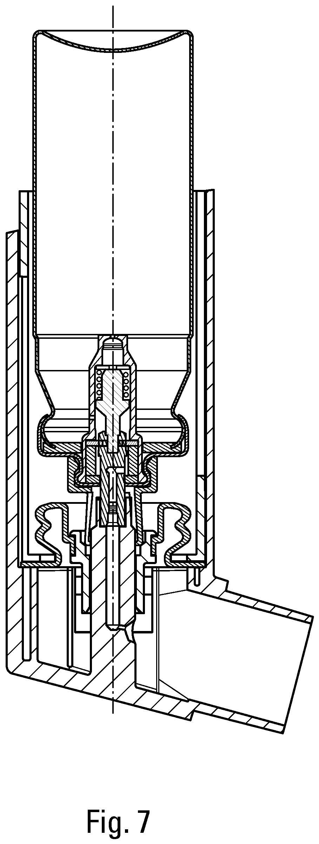

En fin d'inhalation, l'élément déclencheur 600 est ramené vers le haut par l'élasticité de la membrane 61. La figure 7 illustre la position où l'élément déclencheur 600 a été ramené par ladite membrane 61, mais avant que l'utilisateur relâche sa pression sur le réservoir 100.

Lorsque l'utilisateur relâche sa pression sur le fond du réservoir 100, celui-ci revient en direction de la position de repos sous l'effet du ressort de rappel de la valve 200, et simultanément la soupape 210 de la valve doseuse revient en position de repos, en chargeant une nouvelle dose de produit fluide dans la chambre de valve. Le dispositif est alors prêt pour une autre utilisation.

La figure 9 illustre une variante de réalisation du mode de réalisation de la figure 8, embarquant des modules électroniques.

En particulier, il est prévu un compteur de doses électronique 1000, avantageusement assemblé sur le corps 10. Ce compteur 1000 peut notamment détecter les déplacements du réservoir 100, par exemple au moyen d'un capteur de contact 1010. En variante, le compteur 1000 pourrait être relié à un capteur, notamment un capteur à membrane, qui détecte la distribution de la dose de produit fluide, par exemple dans le puits de soupape 700. D'autres moyens d'actionner le compteur électronique 1000 sont aussi possibles, par exemple la détection du déplacement de la soupape 210 de la valve doseuse par rapport au corps de valve 201.

De préférence, le dispositif comporte aussi des moyens d'émission de signaux 1100 pour communiquer à distance notamment des informations relatives aux actionnements du dispositif. En particulier, le corps 10 peut comporter un module d'émission de signaux, pour communiquer à distance avec une base quelconque. Des moyens d'alimentation appropriés sont avantageusement prévus.

Avantageusement, le module électronique peut comprendre notamment une carte comportant un commutateur électrique qui envoie une impulsion. Le module peut également comporter un afficheur et/ou utiliser une connexion Bluetooth ou Wifi pour envoyer les informations sur un périphérique annexe. Des capteurs appropriés peuvent être prévus, tel que des capteurs de débit et/ou de pression, pour détecter divers paramètres du flux d'inhalation.

Associés à un compteur de dose 1000 qui compte chaque dose effectivement émise et au dispositif de synchronisation avec l'inhalation de l'invention, ces moyens d'émission de signaux 1100 permettent de transmettre de manière absolument fiable chaque distribution de dose, par exemple à un médecin ou toute autre personne souhaitant surveiller l'utilisation du dispositif d'inhalation par l'utilisateur. Le dispositif de synchronisation avec l'inhalation garantit que l'utilisateur inhale à chaque fois qu'il actionne le dispositif, et le compteur enregistre chaque dose distribuée, ainsi que différents paramètres associés, tel que l'horodatage de chaque distribution. Le médecin peut ainsi connaitre très exactement les conditions d'utilisation du dispositif par l'utilisateur.

Les figures 14 à 16 illustrent un autre mode de réalisation, dans lequel l'élément d'actionnement est réalisé sous la forme d'un élément de blocage 500', qui, en position de non actionnement, empêche le déplacement axial du réservoir 100 dans le corps 10. Lors de l'inhalation, cet élément de blocage 500' est déplacé et/ou déformé de telle sorte qu'il ne bloque plus le déplacement axial du réservoir 100 dans le corps 10. Ainsi, après inhalation, un tel déplacement axial du réservoir 100 provoque l'actionnement de la valve doseuse 200 et la distribution d'une dose de produit synchronisée avec cette inhalation.

L'organe sensible à l'inhalation est ici réalisé sous la forme d'un piston 65 coulissant dans une chambre 66, entre une position de repos et une position d'inhalation. La chambre 66 est avantageusement formée dans l'embout buccal 400. Une tige 540 est reliée audit piston 65. Un ressort 67, disposé avantageusement dans la chambre 66, est adapté à ramener ledit piston 65 vers sa position de repos lorsqu'il n'y a plus d'inhalation à travers l'embout buccal 400.

Lorsque l'utilisateur inhale à travers l'embout buccal 400, le piston 65 se déplace radialement (par rapport à l'axe de déplacement du réservoir 100 dans le corps 10) dans la chambre 66 sous l'effet de la dépression crée par l'inhalation.

L'élément de blocage 500' peut notamment être assemblé sur ladite tige 540 solidaire du piston 65, et comporter une extension axiale 505 qui, en position de non actionnement s'étend axialement dans le corps pour coopérer avec et bloquer axialement ledit réservoir 100. Lorsque l'utilisateur inhale, la tige 540 se déplace radialement vers la gauche (dans l'orientation des figures), ce qui va provoquer la déformation de ladite extension axiale 505, et donc libérer le déplacement axial du réservoir 100. Dans l'exemple représenté, le puits de soupape 700 est monté mobile dans le corps 10, mais il pourrait aussi être fixe.

Avantageusement, l'extrémité inférieure 506 de ladite extension axiale 505 est fixée radialement et axialement par rapport au corps 10. Ainsi, lorsque la tige 540 se déplace radialement, elle tire radialement sur ladite extension axiale, qui se déforme, par exemple se plie ou pivote, de sorte que l'extrémité supérieure se désengage du réservoir 100 et libère celui-ci pour son déplacement axial. Bien entendu, l'élément de blocage 500' pourrait avoir toute autre forme appropriée. En particulier, on peut envisager l'utilisation d'une genouillère articulée.

La figure 16 représente une variante de réalisation, dans laquelle la tige 540 est accessible de l'extérieur du corps 10 à travers une ouverture 19 dudit corps 10. Ceci permet en cas de nécessité de déplacer manuellement l'élément de blocage 500', pour pouvoir réaliser un actionnement de la valve doseuse 200 même sans inhalation, par exemple si la personne devant recevoir la dose de produit fluide est incapable de réaliser une inhalation suffisante. Il s'agit donc d'une sécurité.

Le dispositif représenté sur les figures 14 à 16 peut comprendre également des moyens électroniques, tels que ceux décrits précédemment en relation avec la figure 9. Ils ne seront donc pas décrits à nouveau ici.

La présente invention est notamment applicable pour le traitement des crises d'asthme ou de BPCO (Broncho Pneumopathie Obstructive) en utilisation avec des formulations de type salbutamol, aclidinium, formoterol, tiotropium, budesonide, fluticasone, indacaterol, glycopyrronium, salmeterol, umeclidinium bromide, vilanterol, olodaterol, striverdi, ou toute combinaison de ces formulations.

La présente invention a été décrite en référence à plusieurs modes de réalisation et variantes avantageux, mais il est entendu qu'un homme du métier peut y apporter toutes modifications, sans sortir du cadre de la présente invention tel que défini par les revendications annexées.

## Revendications

1. Dispositif de distribution de produit fluide synchronisé avec l'inhalation, comportant un corps (10) pourvu d'un embout buccal (400), un réservoir de produit (100) contenant un produit fluide et un gaz propulseur étant monté axialement coulissant dans ledit corps (10), une valve doseuse (200) comportant une soupape (210) étant assemblée sur ledit réservoir (100) pour distribuer sélectivement le produit fluide, ledit dispositif comportant:
- un élément d'actionnement (500, 500') déplaçable et/ou déformable entre une position de non actionnement, dans laquelle ladite valve doseuse (200) ne peut pas être actionnée, et une position d'actionnement, dans laquelle ladite valve doseuse (200) peut être actionnée, et
- un système de déclenchement commandé par l'inhalation comportant un organe sensible à l'inhalation (61; 65), déformable et/ou déplaçable sous l'effet de l'inhalation, ledit organe sensible à l'inhalation (61; 65), lorsqu'il se déforme et/ou se déplace, déplaçant et/ou déformant ledit élément d'actionnement (500, 500') de sa position de non actionnement vers sa position d'actionnement,
ledit élément d'actionnement étant un élément de blocage (500, 500') qui, en position de non actionnement, coopère d'une part avec le corps (10) et d'autre part avec le réservoir (100) pour empêcher le déplacement axial dudit réservoir (100) dans le corps (10), **caractérisé en ce que** ledit élément d'actionnement est une bague de blocage (500) fixée, notamment encliquetée, sur ledit réservoir (100) et comportant au moins une patte axiale (501), notamment trois, coopérant avec un épaulement (710) solidaire dudit corps (10) pour bloquer le déplacement axial dudit réservoir (100) dans ledit corps (10).

2. Dispositif selon la revendication 1, dans lequel ladite au moins une patte axiale (501) est déformable radialement vers l'extérieur pour passer de la position de non actionnement vers la position d'actionnement, un élément déclencheur (600) étant prévu pour maintenir ladite au moins une patte axiale (501) en position de non actionnement.

3. Dispositif selon la revendication 2, dans lequel ledit élément déclencheur (600) est monté déplaçable entre une position de blocage, dans laquelle il bloque ladite bague de blocage (500) dans sa position de non actionnement, et une position de libération, dans laquelle il ne bloque pas ladite bague de blocage (500).

4. Dispositif selon la revendication 3, dans lequel ledit système de déclenchement commandé par l'inhalation comporte une membrane déformable (61) définissant une chambre d'air déformable (60), ladite membrane déformable (61) étant fixée audit élément déclencheur (600), ladite membrane déformable (61) étant déformée lors de l'inhalation de telle sorte qu'elle déplace ledit élément déclencheur (600) de sa position de blocage vers sa position de libération.

5. Dispositif selon l'une quelconque des revendications 2 à 4, dans lequel ledit élément déclencheur (600) est accessible par l'utilisateur à travers au moins une ouverture (13) du corps (10), pour pouvoir être déplacé manuellement vers sa position de libération même en l'absence d'inhalation.

6. Dispositif selon l'une quelconque des revendications précédentes, comportant un compteur de doses électronique (1000).

7. Dispositif selon l'une quelconque des revendications précédentes, comportant des moyens d'émission de signaux (1100) pour communiquer à distance notamment des informations relatives aux actionnements du dispositif.

## Patentansprüche

1. Vorrichtung zur inhalationssynchronisierten Ausgabe von Fluidprodukt, aufweisend einen Körper (10), der mit einem Mundstück (400) versehen ist, einen Produktbehälter (100), der ein Fluidprodukt und ein Treibgas enthält, der axial gleitend in dem Körper (10) gelagert ist, ein Dosierventil (200) mit einem Ventil (210), das an dem Behälter (100) angebracht ist, um das Fluidprodukt selektiv abzugeben, die Vorrichtung aufweisend:
- ein Betätigungselement (500, 500'), das zwischen einer Nichtbetätigungsposition, in welcher das Dosierventil (200) nicht betätigt werden kann, und einer Betätigungsposition, in welcher das Dosierventil (200) betätigt werden kann, bewegbar und/oder verformbar ist, und
- ein Auslösesystem, das durch die Inhalation gesteuert wird, mit einem inhalationsempfindlichen Element (61; 65), das unter der Wirkung der Inhalation verformbar und/oder bewegbar ist, wobei das inhalationsempfindliche Element (61; 65), wenn es sich verformt und/oder bewegt, das Betätigungselement (500, 500') aus seiner Nichtbetätigungsposition zu seiner Betätigungsposition bewegt und/oder verformt,
wobei das Betätigungselement ein Blockierelement (500, 500') ist, das in der Nichtbetätigungsposition einerseits mit dem Körper (10) und andererseits mit dem Behälter (100) zusammenwirkt, um die axiale Bewegung des Behälters (100) im Körper (10) zu verhindern, **dadurch gekennzeichnet, dass** das Betätigungselement ein Blockierring (500) ist, der auf dem Behälter (100) befestigt, insbesondere eingerastet ist, und mindestens einen axialen Ansatz (501) aufweist, insbesondere drei, die mit einem Absatz (710) zusammenwirken, der mit dem Körper (10) fest verbunden ist, um die axiale Bewegung des Behälters (100) im Körper (10) zu blockieren.

2. Vorrichtung nach Anspruch 1, wobei der mindestens eine axiale Ansatz (501) radial nach außen verformbar ist, um aus der Nichtbetätigungsposition zu der Betätigungsposition zu wechseln, wobei ein Auslöseelement (600) vorgesehen ist, um den mindestens einen axialen Ansatz (501) in der Nichtbetätigungsposition zu halten.

3. Vorrichtung nach Anspruch 2, wobei das Auslöseelement (600) zwischen einer Blockageposition, in welcher es den Blockierring (500) in seiner Nichtbetätigungsposition blockiert, und einer Freigabeposition, in welcher es den Blockierring (500) nicht blockiert, bewegbar gelagert ist.

4. Vorrichtung nach Anspruch 3, wobei das Auslösesystem, das durch die Inhalation gesteuert wird, eine verformbare Membran (61) aufweist, die eine verformbare Luftkammer (60) definiert, wobei die verformbare Membran (61) an dem Auslöseelement (600) befestigt ist, wobei die verformbare Membran (61) bei der Inhalation derart verformt wird, dass sie das Auslöseelement (600) aus seiner Blockageposition zu seiner Freigabeposition bewegt.

5. Vorrichtung nach einem der Ansprüche 2 bis 4, wobei das Auslöseelement (600) dem Benutzer über mindestens eine Öffnung (13) des Körpers (10) zugänglich ist, um per Hand zu seiner Freigabeposition bewegt zu werden, selbst ohne Inhalation.

6. Vorrichtung nach einem der vorhergehenden Ansprüche, aufweisend einen elektronischen Dosiszähler (1000).

7. Vorrichtung nach einem der vorhergehenden Ansprüche, aufweisend Signalsendemittel (1100) zur Fernkommunikation insbesondere von Informationen im Zusammenhang mit den Betätigungen der Vorrichtung.

## Claims

1. An inhalation-synchronized fluid dispenser device comprising a body (10) provided with a mouthpiece (400), a fluid reservoir (100) containing a fluid and a propellant gas being mounted to slide axially in said body (10), a metering valve (200) including a valve member (210) being assembled on said reservoir (100) for selectively dispensing the fluid, said device further comprising:
• an actuator element (500, 500') that is movable and/or deformable between a non-actuation position, in which said metering valve (200) cannot be actuated, and an actuation position in which said metering valve (200) can be actuated, and
• an inhalation-controlled trigger system including an inhalation-sensitive member (61, 65), that is deformable and/or movable under the effect of inhaling, said inhalation-sensitive member (61, 65), when it is deformed and/or moved, moving and/or deforming said actuator element (500, 500') from its non-actuation position towards its actuation position,
said actuator element being a blocking element (500, 500') that, in the non-actuation position, co-operates firstly with the body (10), and secondly with the reservoir (100) so as to prevent said reservoir (100) from moving axially in the body (10), the dispenser device being **characterized in that** said actuator element is a blocking ring (500) that is fastened, in particular snap-fastened, on said reservoir (100), and that includes at least one axial tab (501), in particular three axial tabs, that co-operate with a shoulder (710) that is secured to said body (10) so as to prevent said reservoir (100) from moving axially in said body (10).

2. A device according to claim 1, wherein said at least one axial tab (501) is deformable radially outwards so as to pass from the non-actuation position towards the actuation position, a trigger element (600) being provided so as to hold said at least one axial tab (501) in the non-actuation position.

3. A device according to claim 2, wherein said trigger element (600) is mounted to move between a blocking position in which it blocks said blocking ring (500), in its non-actuation position, and a release position, in which it does not block said blocking ring (500).

4. A device according to claim 3, wherein said inhalation-controlled trigger system includes a deformable membrane (61) that defines a deformable air chamber (60), said deformable membrane (61) being fastened to said trigger element (600), said deformable membrane (61) being deformed during inhaling, so that it moves said trigger element (600) from its blocking position towards its release position.

5. A device according to any one of claims 2 to 4, wherein said trigger element (600) is accessible to the user through at least one opening (13) in the body (10), so that it can be moved manually towards its release position even in the absence of inhaling.

6. A device according to any preceding claim, including an electronic dose counter (1000).

7. A device according to any preceding claim, including signal-transmitter means (1100) for communicating, in particular communicating remotely, information relating to the actuations of the device.
